**Europäisches Patentamt**

**European Patent Office**

**Office européen des brevets**

(19)

(11) Numéro de publication: **0 123 594
B1**

# FASCICULE DE BREVET EUROPEEN

(12)

(45) Date de publication du fascicule du brevet:
**19.11.87**

(51) Int. Cl.⁴: **A 61 B 1/00**

(21) Numéro de dépôt: **84400677.5**

(22) Date de dépôt: **05.04.84**

(54) Sonde endoscopique de visualisation et d'échographie ultrasonore à balayage.

(30) Priorité: **06.04.83 FR 8305610**

(43) Date de publication de la demande:
**31.10.84 Bulletin 84/44**

(45) Mention de la délivrance du brevet:
**19.11.87 Bulletin 87/47**

(84) Etats contractants désignés:
**AT BE CH DE FR GB IT LI LU NL SE**

(56) Documents cités:
**EP - A - 0 008 517
EP - A - 0 039 851
EP - A - 0 061 331
EP - A - 0 066 185
EP - A - 0 077 923
EP - A - 0 092 080
EP - A - 0 094 791
DE - A - 3 219 118
FR - A - 2 173 115
FR - A - 2 507 075
US - A - 3 936 791
US - A - 4 217 516**

(73) Titulaire: **Universite Francois Rabelais, 2 bis, Boulevard Tonnelé, F-37032 Tours (FR)**

(72) Inventeur: **Pourcelot, Léandre, 14, rue Georges Guynemer, F-37170 Saint Avertin (FR)**
Inventeur: **Fleury, Gérard, La Charmoise Rue Jean Inglessi, F-37230 Fondettes par Luynes (FR)**
Inventeur: **Berson, Marceau, 11, rue de Bourgueil, F-37300 Joue Les Tours (FR)**

(74) Mandataire: **Fort, Jacques et al, CABINET PLASSERAUD 84, rue d'Amsterdam, F-75009 Paris (FR)**

## Description

L'invention a pour objet une sonde endoscopique de visualisation et d'échographie ultrasonore et elle trouve une application particulièrement importante, bien que non exclusive, dans le domaine médical, constituée par l'observation optique et en échographie des parois internes du corps humain, notamment de l'œsophage, de l'estomac et du duodenum.

On utilise déjà largement des endoscopes optiques destinés à l'observation des parois internes du corps humain. Ces endoscopes sont constitués par un long tuyau souple fixé à une extrémité sur une boîte de commande comprenant des organes de guidage, par câbles fins, d'un tronçon terminal rigide du tuyau. La lumière est transmise depuis une source externe jusqu'à un émetteur, placé dans le tronçon terminal et destiné à illuminer la paroi, par des fibres optiques passant dans le tuyau. L'image optique recueillie par un objectif situé à côté de l'émetteur est transmise par fibres optiques jusqu'à un œilleton de visée porté par la boîte.

On a déjà proposé (EP-A-00 66 185) de compléter un tel endoscope par des moyens d'échotomographie des tissus et organes situés derrière les parois observées optiquement. Pour cela, on implante un transducteur ultrasonore dans le tronçon terminal, à côté du système optique. Les deux modes d'exploration sont complémentaires: l'image optique permet le guidage et la localisation du transducteur ultrasonore qui est utilisé pour fournir l'image acoustique. Cette dernière peut être de bonne qualité en utilisant des fréquences élevées, du fait de la faible profondeur à explorer et de l'absence d'interposition de tissus fortement absorbants entre le transducteur et l'organe.

L'endoscope à échotomographie décrit dans le document EP-A-0066 185 utilise un seul transducteur, muni d'un système de balayage mécanique permettant de le faire osciller autour d'un axe confondu avec celui du tronçon terminal. Cette technique présente, entre autres inconvénients, des difficultés de réalisation des pièces mécaniques en mouvement relatif et des difficultés de repérage du mouvement du transducteur. Il en est de même lorsque ce dernier est fixe et que l'orientation du faisceau ultrasonore est modifiée à l'aide d'un miroir tournant.

Le document EP-A-0 077 923, publié après la date de priorité de la présente demande, fait connaître une sonde endoscopique qui comporte un réseau linéaire de transducteur autorisant un balayage électronique et un bloc optique d'éclairage et d'observation depuis l'extrémité proximale de la sonde, ce bloc optique débouchant par une face inclinée sur l'axe de l'endoscope, immédiatement en arrière du réseau de transducteur, de sorte que son champ de vision présente un recouvrement avec le champ de balayage du transducteur.

L'invention vise à fournir une sonde endoscopique de visualisation et d'échographie ultrasonore répondant mieux que celles antérieurement connues aux exigences de la pratique, notamment en ce qu'elle fournit un grand champ angulaire acoustique sans mettre en œuvre des pièces mobiles, une résolution élevée et une possibilité de guidage optique, tout en conservant des dimensions comparables à celles des sondes classiques.

Dans ce but, l'invention propose notamment une sonde endoscopique comprenant un organe tubulaire destiné à être inséré dans une cavité à explorer, présentant une partie souple munie d'un tronçon terminal orientable à l'aide de moyens mécaniques de commande disposés à l'intérieur de la partie souple, des moyens optiques d'illumination et de retour d'image, et un transducteur électro-acoustique porté par ledit tronçon et comprenant un réseau linéaire d'éléments piézo-électriques fixés dans le tronçon et associés à un circuit électronique d'excitation des éléments suivant une séquence de balayage dans un champ présentant un recouvrement avec le champ de vision des moyens optiques, caractérisée en ce que le réseau est courbe, la convexité de la courbe étant dirigée vers l'extérieur, et en ce que le circuit électronique comporte des moyens de retard électrique et/ou de déphasage, permettant de réaliser un balayage électronique sectoriel dans un plan passant par l'axe du tronçon terminal de la sonde par excitation de groupes successifs d'éléments piézo-électriques avec focalisation à distance déterminée du réseau.

Le réseau linéaire sera généralement constitué par une seule ligne d'éléments piézo-électriques, le balayage sectoriel s'effectuant dans un plan passant par l'axe du tronçon terminal. Le circuit électronique comportera généralement des moyens de retard électrique et/ou de déphasage permettant de réaliser non seulement un balayage électronique par excitation de groupes successifs d'éléments, mais aussi une focalisation à distance déterminée du réseau. Au balayage sectoriel réalisé grâce à la forme convexe du réseau peut s'ajouter un balayage obtenu par modification de la répartition des phases et/ou retards au cours de tirs successifs à partir d'un même groupe d'éléments piézoélectriques, suivant une technique comparable à celle utilisée dans les réseaux plans phasés.

La réalisation matérielle de la sonde pourra prendre des formes très différentes suivant l'application envisagée. On décrira par la suite, à titre d'exemples, des modes de réalisation destinés au domaine médical, mais une sonde du même genre peut être utilisée dans de nombreux domaines industriels, pour explorer des régions de machines ou d'installations inaccessibles sans démontage.

L'invention sera mieux comprise à la lecture de la description qui suit de sondes qui en constituent des modes partiucliers de réalisation, donnés à titre d'exmples non limitatifs. La description se réfère aux dessin qui l'accompagnent, dans lesquels:

– la Figure 1 est un schéma de principe montrant la constitution générale d'une sonde suivant l'invention, en élévation et en coupe partielle,

– la Figure 2 montre, à grande échelle, l'émetteur de lumière et l'objectif porté par le tronçon terminal de la sonde, vus suivant la direction de la flèche II des Figures 1 et 3,

– la Figure 3 est une vue à grande échelle du tronçon terminal de la sonde, en coupe suivant un plan passant par le plan de symétrie,

– la Figure 4 est une vue en coupe suivant la ligne IV–IV des Figures 1 et 3,

– les Figures 5, 6 et 7 sont des schémas destinés à faire apparaître des modes de balayage acoustique utilisables dans la mise en œuvre de la sonde,

– la Figure 8 est un synoptique montrant une constitution possible de l'électronique de la sonde,

– les Figures 9, 10 et 11 sont des schémas montrant diverses variantes possibles de réalisation du tronçon terminal de la sonde.

La sonde endoscopique qui sera maintenant décrite est notamment destinée à permettre l'étude d'organes et de zones du corps humain difficiles d'accès par voie externe, tels que les artères coronaires, l'aorte descendante, le cœur droit, les oreillettes, les masses médiastinales, le pancreas, les voies biliaires basses et les vaisseaux digestifs.

La sonde comprend un organe tubulaire d'insertion 11 relié à une boîte 12 de commande et de connexion. L'organe tubulaire 11 présente une partie souple 13 en forme de tuyau et un tronçon terminal 14 qui sera généralement rigide. La partie souple contient les différents conducteurs électriques et de lumière nécessaires pour alimenter les éléments qui se trouvent dans le tronçon terminal 14. Elle contient également des câbles fins 15 de guidage et d'orientation du tronçon terminal 14, câbles fixés deux à deux à leurs extrémités sur des anneaux 16 noyés dans la paroi de la partie souple, à proximité immédiate du tronçon terminal. Ces câbles, au nombre de quatre dans le mode de réalisation illustré, sont reliés à des boutons de commande 17 portés par la boîte 12.

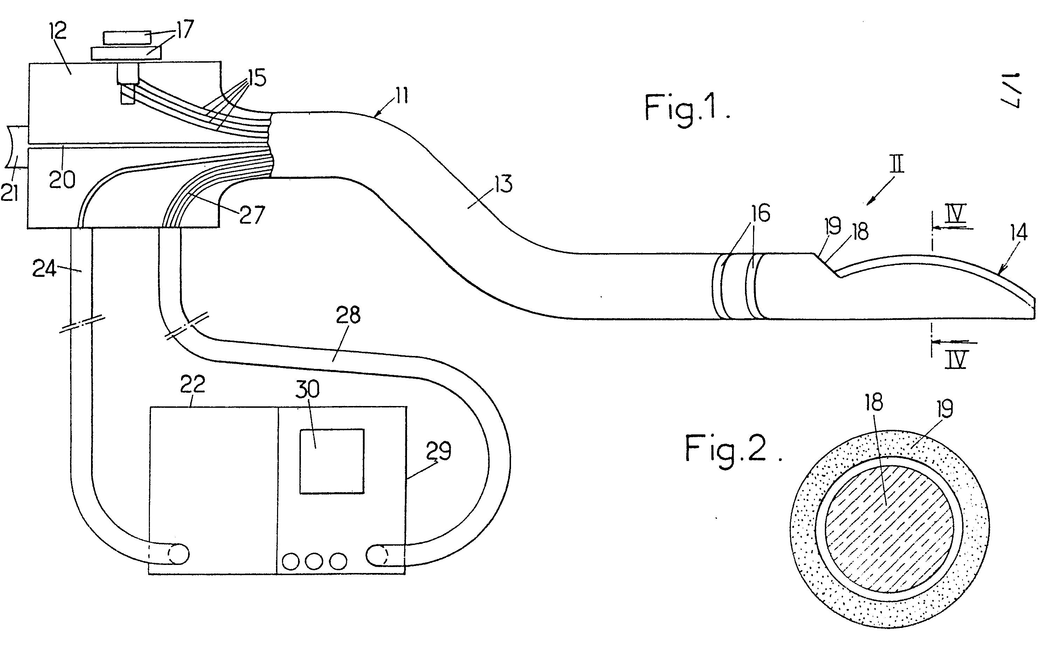

La partie optique de la sonde comporte un objectif 18 et un émetteur de lumière 19 prévus sur le tronçon terminal 14 (Figure 2), sur une paroi oblique de façon à ce qu'ils soient écartés de la paroi et, comme on le verra plus loin, présentent un champ de vision ayant un recouvrement angulaire avec celui des moyens d'échographie. L'objectif 18 est relié, par un guide optique souple 20, contenu dans l'organe tubulaire 11, à un œilleton d'observation 21. L'émetteur de lumière 19 qui, dans le mode de réalisation illustré, est de forme annulaire et entoure l'objectif 18, reçoit un flux lumineux provenant d'une source 22 par une fibre optique 23 qui suit successivement un tuyau 24 de raccordement entre la source 22 et la boîte 12 et l'organe tubulaire 11.

Les éléments de la sonde destinés à l'exploration ultrasonore comprennent un transducteur ultrasonore 25 constituant une partie de la paroi latérale du tronçon terminal rigide 14, un circuit imprimé 26 contenu dans ce tronçon terminal et des câbles électriques coaxiaux miniatures 27 reliant le circuit 26, à travers la boîte 12 et une gaine 28 à coffret électronique 29 portant un écran de visualisation 30 et des boutons de mise en route et de réglage non représentés.

L'élément essentiel du transducteur ultrasonore 25 est un réseau 31 de N éléments électro-acoustiques, disposés suivant un arc de cercle de rayon R à convexité tournée vers l'extérieur. A titre d'ordre de grandeur, on peut indiquer qu'une rangée de N=64 à 104 éléments (plaquettes de matériau piézo-électriques) disposés suivant une seule rangée, suivant un arc de cercle de rayon R=30 mm, avec une corde D=30 mm, donnera des résultats satisfaisants. La longueur de chaque élément, qui correspond à la largeur du transducteur, pourra être de 0,5 cm, la fréquence de résonance pouvant aller de 5 à 10 MHz.

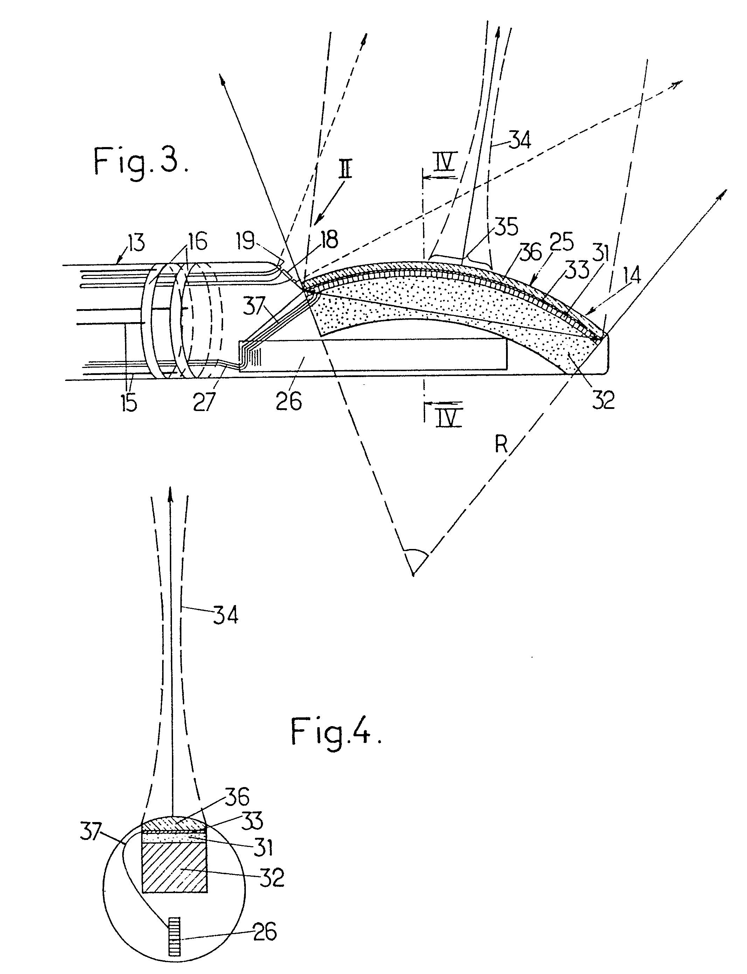

Les éléments piézo-électriques sont portés, de façon classique, par une masse 32 de matériau amortisseur destiné à absorber l'énergie émise par les éléments vers l'arrière et à amortir rapidement les vibrations de ces éléments, afin d'élargir la bande passante et d'optimiser la résolution axiale. Sur la face avant du réseau d'éléments piézo-électriques est collée une lame adaptatrice 33, d'épaisseur égale au quart de la longueur d'onde des ultrasons dans le matériau qui la constitue. Enfin, pour focaliser le faisceau acoustique 34 émis par un groupe d'éléments 35 au cours d'un même tir, dans le sens transversal au plan de symétrie, une lentille acoustique 36 est collée sur la lame adaptatrice 33. On constitue avantageusement la lentille 36 d'un matériau dans lequel la vitesse de propagation est inférieure à la vitesse dans les tissus (1500 m/s environ). On choisira par exemple un matériau dans lequel la vitesse de propagation est d'environ 1000 m/s. Ainsi, la focalisation est obtenue avec une forme convexe de la lentille acoustique 36 (Figure 4). La face externe de la lentille plan convexe sera très peu différente de la courbure du tronçon terminal 14 de l'endoscope, ce qui assure une bonne adaptation et un bon contact avec la paroi de la cavité à explorer.

Le circuit imprimé 26, de faible dimension, permet d'éviter les problèmes de connexion directe de câbles coaxiaux sur les éléments du réseau 31. Les câbles coaxiaux sont en effet reliés au circuit 26, connecté aux éléments par des fils minces et courts 37 qui transportent les impulsions électriques d'excitation des éléments piézo-électriques et ramènent les informations électriques d'écho.

Le balayage électronique, faisant intervenir des groupes successifs d'éléments piézo-électriques du réseau 31, est réalisé par l'un quelconque des procédés actuellement bien connus, en décalant pas à pas le groupe 35 d'éléments participant à un même tir. La focalisation des faisceaux successifs 34 est également obtenue de façon en elle-même connue, éventuellement à profondeur variable, en utilisant un ensemble de retards tenant compte de la profondeur de focalisation à obtenir et de la courbure du réseau. Grâce à la combinaison d'une focalisation électronique dans le sens du balayage et d'une focalisation acoustique dans le plan perpendiculaire, on peut arriver à une résolution élevée et à un champ beaucoup plus important qu'avec une barrette plan d'éléments piézo-électriques.

Comme le montre la Figure 3, le système optique et le système d'échographie ultrasonore sont avantageusement disposés de façon que le champ optique et le champ acoustique aient une région commune importante, dont l'étendue angulaire est proche de 80° dans le mode de réalisation illustré. Ce recouvrement a l'intérêt de permettre une visualisation optique de la paroi sur laquelle est appliqué le capteur acoustique et, donc, une vérification des conditions du contact, favorisé par ailleurs par la forme courbe du réseau. Il faut en particulier remarquer que l'on peut appliquer de façon étroite le tronçon terminal contre la paroi en agissant sur les câbles de guidage 15.

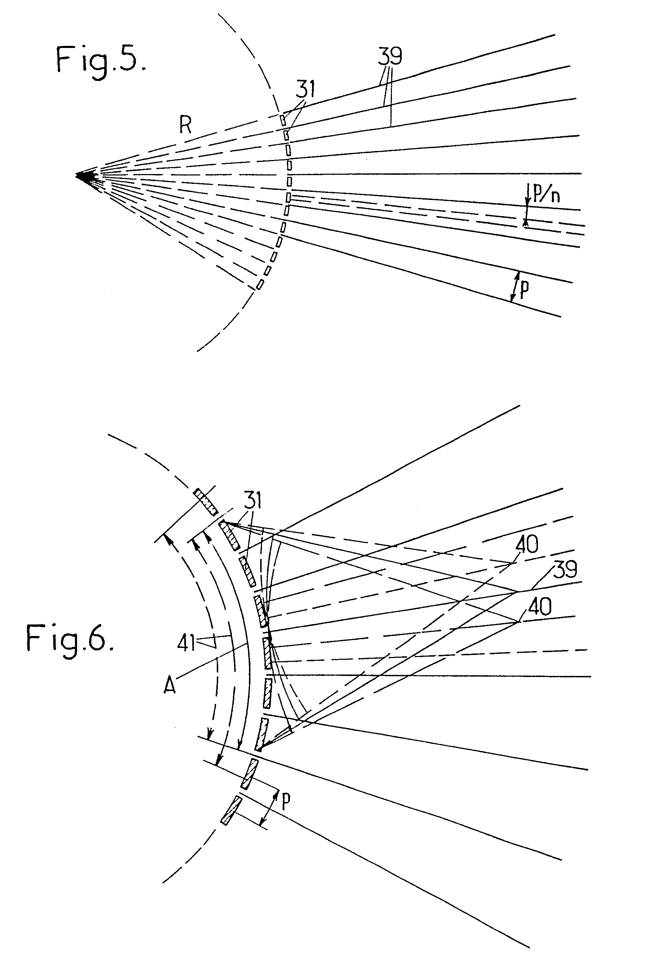

La Figure 5 montre que le balayage sectoriel avec un réseau linéaire courbe d'éléments électro-acoustiques se traduit par un écartement entre les lignes d'exploration successives 39 qui augmente avec la profondeur et peut devenir excessif à distance du transducteur. Pour réduire cet écartement, qui est fonction du pas $p$ de répartition des éléments 31, une solution consiste à effectuer plusieurs tirs successifs à partir du même groupe d'éléments 31, mais avec des répartitions de retard différentes pour effectuer un balayage avec un pas $p/n$ ($n$ étant égal à 2 ou 3). Ce procédé permet d'ajouter $(n-1)$ lignes d'exploration, indiquées en tirets sur la Figure 5, entre deux lignes normales d'exploration 39 adjacentes. Comme le montre la Figure 6 dans le cas d'un groupe de six éléments électro-acoustiques 31 et de $n=3$, on obtient une ligne d'exploration normale avec une répartition symétrique des retards et deux focalisations suivant des lignes additionnelles 40, symétriques par rapport à la ligne 39, avec des retards correspondant à deux ouvertures ficitives 41 égales à $A + \dfrac{2p}{n}$. Ce mode de mise en œuvre provoque une division de la cadence de répétition de l'image par $n$, mais cette réduction est peu gênante pour les profondeurs d'exploration habituelles, ne dépassant pas 6 cm. Ainsi, pour $n=3$ et un transducteur 25 comportant un réseau de 100 éléments 31, la cadence est de 60 images par seconde pour une profondeur d'exploration de 4 cm, ce qui est très supérieur à la limite de scintillement, d'environ 24 images par seconde.

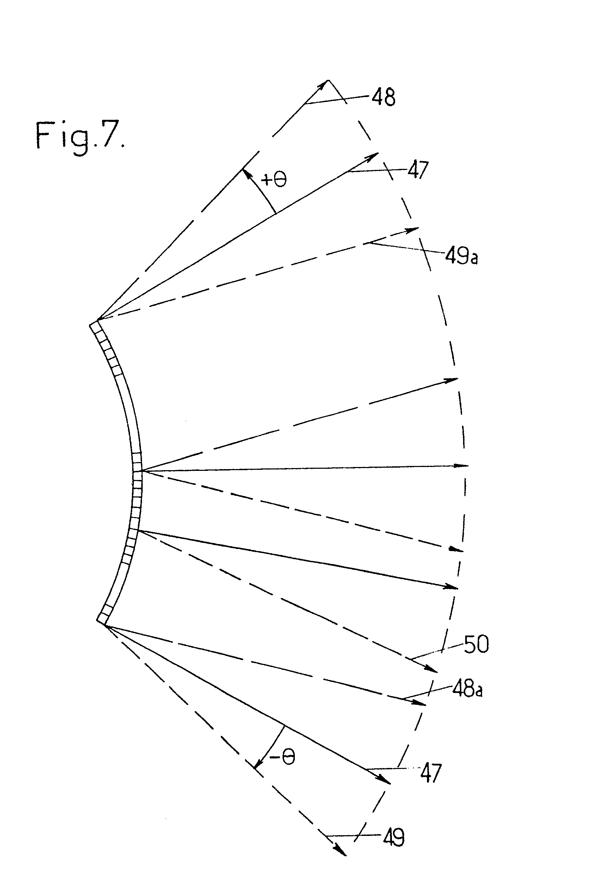

On peut incliner le faisceau acoustique par rapport à la normale à l'élément médian. La Figure 7 montre par exemple les lignes d'exploration terminales 47 du champ normal, ainsi que les limites d'exploration 48 avec une inclinaison $+\ominus$ et 49 avec une inclinaison $-\ominus$. La déflexion par voie électronique est obtenue de la même façon qu'avec un réseau plan phasé, souvent désigné par la formulation anglo-saxonne «phased array», à l'aide d'un choix de retards qui assurent la focalisation du faisceau, son inclinaison et la compensation de la courbure du réseau.

L'inclination électronique du faisceau peut être utilisé pour réaliser une focalisation au même point à partir de plusieurs groupes différents d'éléments électro-acoustiques, ce qui permet d'obtenir un balayage composé augmentent le nombre d'informations. Une reconstitution par calculateur permet de mieux cerner les structures arrondies qu'avec une information unique et enrichit considérablement l'image. Avec un tel mode de balayage, l'image résultante résulte de la superposition ou de la combinaison de trois images correspondant respectivement aux champs 47–47, 48, 48a et 49a–49. On peut naturellement arrêter le balayage électronique sur un groupe donné d'éléments électro-acoustiques pour enregistrer le mouvement d'une structure, par exemple en cardiologie. Cette même observation d'une zone fixe permet également de déterminer les écoulements, par exemple l'écoulement sanguin, par détermination de l'effet Doppler, qui s'accomode particulièrement bien d'une incidence oblique telle que celle indiquée en 50.

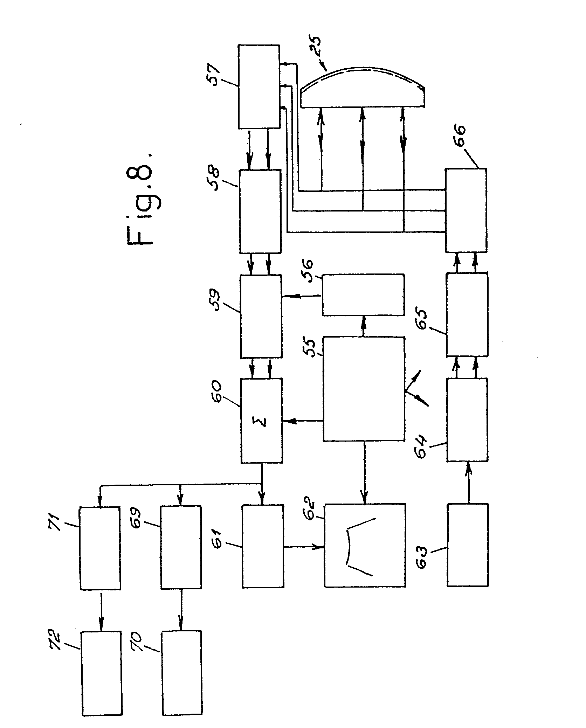

On ne décrira pas ici de façon détaillée l'électronique associée au transducteur 25, étant donné qu'elle peut prendre de nombreuses formes, et notamment celles déjà bien connues. On indiquera simplement, en faisant référence à la Figure 8, une constitution générale possible. Cette électronique comprend un module de commande 55 qui gère les chaînes d'émission et de réception.

Dans la chaîne d'émission, un cadenceur 63 fournit, à intervalles réguliers, une impulsion qui est transmise à un dispositif 64 de stockage des retards correspondant à la focalisation recherchée. Le dispositif 64 fournit les signaux d'excitation présentant des retards différents nécessaires pour la focalisation et, éventuellement, la formation des lignes d'exploration additionnelles. Les signaux provenant du dispositif 64 sont aiguillés par le sélecteur 65 vers les entrées des circuits d'émission 66 dont les sorties délivrent des impulsions d'excitation, de niveau suffisant, aux $x$ éléments électro-acoustiques simultanément excités, d'ordre $n$ à $n+x-1$. Ce groupe d'éléments 35 émet un faisceau ultrasonore focalisé dans les tissus à étudier. Lors de l'émission suivante, les $x$ signaux sont transmis cette fois aux éléments $n+1$ jusqu'à $(n+1)+(x-1)$ et ainsi de suite.

A la réception, le processus est inversé: les $x$ signaux fournis par les $x$ éléments électro-acoustiques 35 du groupe sélectionné par le module de sélection 57 sont transmis à des préamplificateurs 58, puis à un système de retard ou de déphasage 59 et, enfin, à un circuit sommateur 60. Un amplificateur video 61 de gain réglable fournit à un moniteur de visualisation 62 les signaux analogiques nécessaires. Un dispositif 56 sera généralement prévu pour corriger le gain en fonction de la profondeur d'exploration. Cette correction de gain peut être effectuée par un circuit agissant sur les préamplificateurs 58 et commandé par le module 55.

Le signal utile apparaissant à la sortie du sommateur 60 peut être également dirigé vers un dispositif 71 de mesure de l'effet Doppler, suivi d'un analyseur de spectre 72 fonctionnant en temps réel et fournissant le spectre de fréquence Doppler. Ce même signal utile peut être traité dans un dispositif déterminant le mouvement en fonction du temps 69 pour mémorisation sur un enregistreur 70.

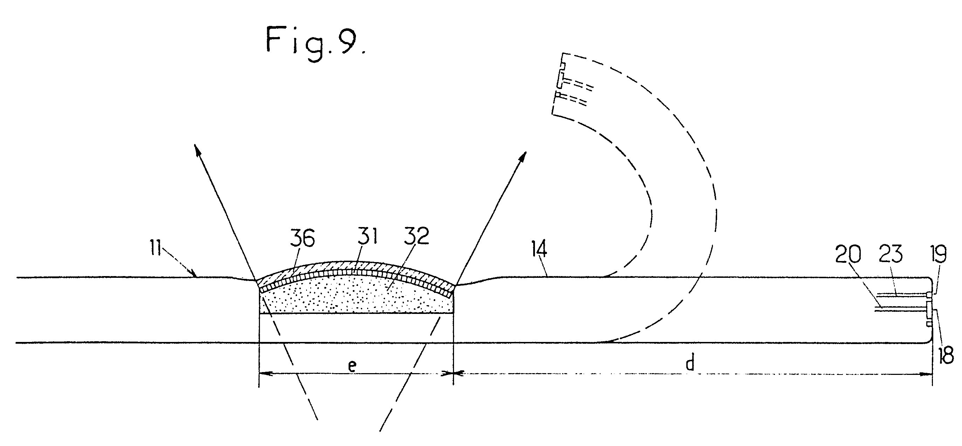

Comme on l'a indiqué plus haut, l'invention est susceptible de nombreuses variantes de réalisation. La Figure 9 montre, en coupe longitudinale, la partie terminale de l'organe tubulaire 11 d'un endoscope à tronçon terminal déformable. Ce tronçon terminal porte encore un transducteur ultrasonore constitué par un réseau courbe 31 d'éléments électro-acoustiques recouvert par une lentille 36 mais le réseau est placé à une distance d de l'extrémité de l'organe tubulaire 11 et le système optique, formé par l'objectif 18 et l'émetteur 19 et sur la tranche terminale du tronçon 14. Le tronçon de l'organe tubulaire 11 contenant le réseau d'éléments électro-acoustiques 31 est rigide, sur une longueur e. Au contraire, la partie de longueur d, généralement d'environ 8 cm, est flexible et orientable. Le guidage peut ainsi être très souple, la partie rigide étant rejetée en arrière de la tranche terminale. De plus, la visualisation optique de la zone de contact entre la face avant du transducteur et la paroi interne est possible lorsque le bout de l'organe tubulaire 11 est amené dans la position montrée en tirets sur la Figure 9.

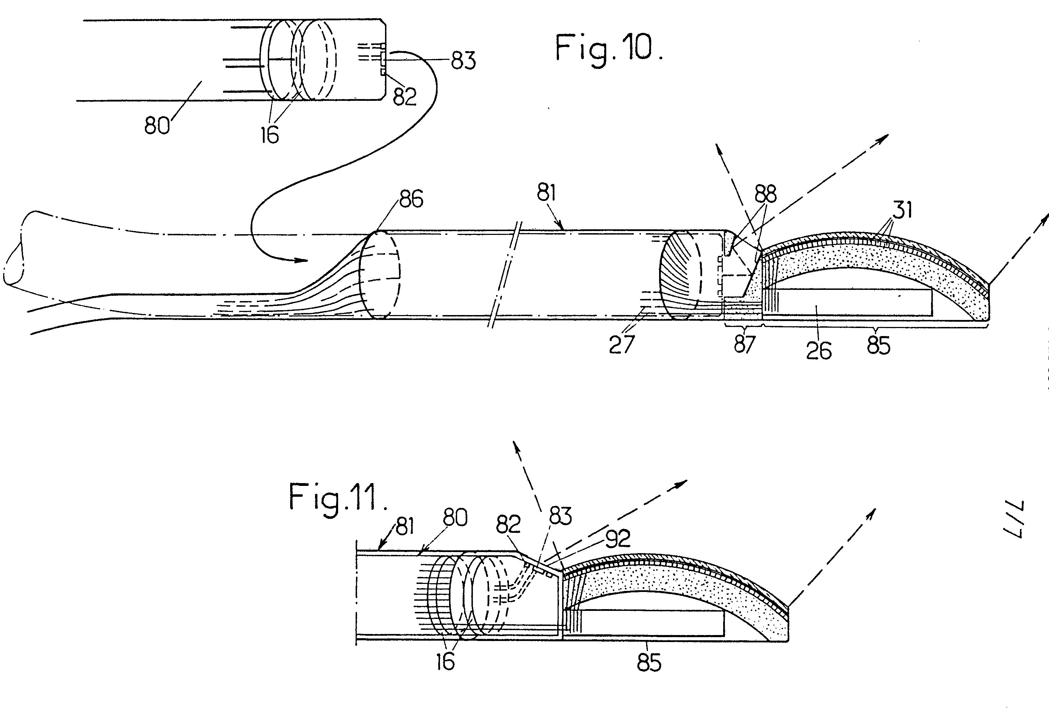

Dans une autre variante encore de réalisation, montrée en Figure 10, l'organe tubulaire 11 est en deux modules séparés et emboîtables 80 et 81. Le module 80 est similaire à un endoscope classique, muni d'un émetteur de lumière 82 et d'un objectif optique 83 sur sa tranche terminale. Le module 81 porte une sonde acoustique 85 du même genre que celle représentée sur la Figure 3. Les câbles électriques 27 de connexion entre les éléments électroacoustiques du réseau et le coffret électronique associé (non représenté) sont répartis dans une gaine cylindrique mince et souple 86 dont le diamètre est légèrement supérieur à celui de l'endoscope 80 et dont la longueur est au moins égale à celle du tuyau de l'endoscope.

Dans la partie 87 du module 82, qui est accolée à la tranche terminale de la sonde acoustique 85, sont prévus deux miroirs 88 ayant une inclinaison telle qu'ils guident la lumière provenant de l'émetteur 82 vers l'extérieur, à travers la partie 87. Ces miroirs permettent également la vision de l'extérieur par l'objectif 83.

Lorsqu'on veut effectuer une exploration échoendoscopique, l'endoscope 83 est d'abord introduit dans la gaine 86 jusqu'à ce que les éléments optiques 82 et 83 soient en regard des miroirs de la partie 87, comme indiqué en traits mixtes sur la Figure 10. Le dispositif de guidage comportant les anneaux 16 permet d'orienter la tête d'échoendoscope formée par la gaine 86, la sonde 85 et l'endoscope 80. Le supplément de longueur imposé au tronçon terminal rigide par l'adjonction de la partie 87 est faible (moins d'un centimètre) et, à ce prix, on peut utiliser un endoscope ordinaire, non modifié. De plus, les modules 80 et 81 peuvent être employés séparément pour des examens échographiques ou endoscopiques classiques.

Le dispositif dont un fragment est montré en Figure 11 constitue une variante de celui de la Figure 10. Il comprend encore un module endoscopique 80 et un module acoustique 81 formé d'une gaine 86 et d'une sonde 85. Mais cette variante ne comporte plus la partie rigide munie de miroirs que l'on trouve sur la Figure 10. En contrepartie, les éléments optiques 82 et 83 ne sont plus placés en bout de l'endoscope, mais latéralement sur une surface inclinée par rapport à l'axe du tuyau. Ces éléments optiques se trouvent donc ainsi en regard d'une membrane 92 lorsque l'endoscope est introduit à fond dans la gaine 86. Au prix d'une légère modification d'un endoscope ordinaire, on évite la complication entraînée par l'adjonction des miroirs dans le cas de la Figure 10.

L'invention est susceptible de nombreuses variantes encore de réalisation, tenant compte notamment de l'application envisagée et il doit être entendu que la portée du présent brevet s'étend à toute variante restant dans le cadre des équivalences.

**Revendications**

1. Sonde endoscopique comprenant un organe tubulaire destiné à être inséré dans une cavité à explorer, présentant une partie souple munie d'un tronçon terminal orientable à l'aide de moyens mécaniques de commande disposés à l'intérieur de la partie souple, des moyens optiques (18, 19, 20, 23) d'illumination et de retour d'image, et un transducteur électro-acoustique (25) porté par ledit tronçon et comprenant un réseau linéaire (31) d'éléments piézo-électriques fixés dans le tronçon et associés à un circuit électronique d'excitation des éléments suivant une séquence de balayage dans un champ présentant un recouvrement avec le champ de vision des moyens optiques, caractérisée en ce que le réseau est courbe, la convexité de la courbe étant dirigée vers l'extérieur, et en ce que le circuit électronique comporte des moyens de retard électrique et/ou de déphasage, permettant de réaliser un balayage électronique sectoriel dans un plan passant par l'axe du tronçon terminal (14) de la sonde par excitation de groupes successifs d'éléments piézo-électriques avec focalisation à distance déterminée du réseau.

2. Sonde suivant la revendication 1, caractérisée en ce que le circuit électronique comporte des moyens de modification de la répartition des phases et/ou retards au cours de tirs successifs à partir d'un même groupe d'éléments piézo-électriques, afin de réaliser des explorations suivant des lignes additionnelles aux lignes normales aux éléments.

3. Sonde suivant la revendication 1 ou 2, caractérisée en ce que les moyens optiques sont disposés sur l'extrémité du tronçon terminal, flexible et orientable, au-delà dudit transducteur électroacoustique et en ce que les moyens mécaniques sont prévus pour donner au tronçon terminal, lors de l'emploi de l'endoscope, une forme courbe telle qu'il y ait recouvrement du champs de vision des moyens optiques et du champs angulaire de balayage sectoriel du transducteur.

4. Sonde selon la revendication 1 ou 2, caractérisée en ce qu'elle est constituée de deux modules (80, 81) séparés et emboitables dont l'un est constitué par un endoscope muni desdits moyens optiques, insérable dans une gaine 86 appartenant à l'autre module qui se termine par ledit tronçon terminal.

5. Sonde selon la revendication 4, caractérisée en ce que l'émetteur de lumière (82) et l'objectif (83) de l'endoscope (80) sont disposés sur la tranche distale de ce dernier et en ce que ledit tronçon terminal comporte des miroirs placés en arrière dudit transducteur et destinés à permettre l'observation à l'aide de l'endoscope.

6. Sonde selon la revendication 4, caractérisée en ce que l'objectif (83) et l'émetteur optique (82) de l'endoscope sont placés à l'extrémité distale du module endoscopique, latéralement et sur une surface inclinée par rapport à l'axe de l'endoscope.

7. Sonde selon l'une quelconque des revendications précédentes, caractérisée en ce que ledit tronçon terminal contient un circuit imprimé (26) relié par des fils fins de jonctions (37) aux éléments piézo-électriques du transducteur (25) et par des câbles coaxiaux miniatures à un coffret électronique relié à l'extrémité de l'organe tubulaire éloignée du tronçon terminal.

8. Sonde selon l'une quelconque des revendications précédentes, caractérisée en ce que le transducteur comporte une lentille acoustique convexe de focalisation dans le sens transversal au plan de symétrie de la sonde, constituée d'un matériau dans lequel la vitesse de propagation des ultrasons est inférieure à celle dans les tissus biologiques.

**Patentansprüche**

1. Endoskopische Sonde mit einem rohrförmigen Organ, das zum Einführen in einen zu untersuchenden Hohlraum bestimmt ist und einen biegsamen Teil aufweist, der mit einem Endstück versehen ist, welches mit Hilfe von im Inneren des biegsamen Teiles angeordneten mechanischen Steuermitteln orientierbar ist, sowie mit optischen Mitteln (18, 19, 20, 23) zur Beleuchtung und Bildwiedergabe und einem elektroakustischen Wandler (25), der am Endstück angebracht ist und ein lineares System (31) von piezoelektrischen Elementen umfasst, die am Endstück befestigt und mit einer elektronischen Erregerschaltung versehen sind, welche die Elemente im Sinne einer Abtastung eines das Gesichtsfeld der optischen Mittel überlappenden Feldes erregt, dadurch gekennzeichnet, dass das System gekrümmt ist, wobei die konvexe Seite der Krümmung nach aussen gerichtet ist, und dass die elektronische Schaltung Mittel zur Verzögerung und/oder Phasenverschiebung aufweist, welche durch Erregung aufeinanderfolgender Gruppen von piezoelektrischen Elementen unter Fokussierung des Systems auf eine vorgegebene Entfernung die Durchführung einer sektoriellen elektronischen Abtastung in einer durch die Achse des Endstückes (14) der Sonde hindurchgehenden Ebene ermöglichen.

2. Sonde nach Anspruch 1, dadurch gekennzeichnet, dass die elektronische Schaltung Mittel zum Ändern der Verteilung der Phasen und/oder Verzögerung im Verlauf der aufeinanderfolgenden Impulsgaben, ausgehend von der gleichen Gruppe von piezoelektrischen Elementen aufweist, um Untersuchungen längs zusätzlicher Linien zu den normalen Linien dieser Elemente zu ermöglichen.

3. Sonde nach Anspruch 1 oder 2, dadurch gekennzeichnet, dass die optischen Mittel am Ende eines biegsamen und orientierbaren Endstückes jenseits des akustischen Wandlers angeordnet sind und dass mechanische Mittel vorgesehen sind, welche dem Endstück beim Gebrauch des Endoskopes eine gekrümmte Form erteilen, um das Gesichtsfeld der optischen Mittel in Deckung mit dem Winkelfeld der sektoriellen Abtastung durch den Wandler zu bringen.

4. Sonde nach Anspruch 1 oder 2, dadurch gekennzeichnet, dass sie aus zwei getrennten und zusammensetzbaren Modulen (80, 81) besteht, von welchen der eine ein mit den optischen Mitteln ausgestattetes Endoskop ist, das in eine Hülse (86) einführbar ist, welche dem anderen, mit dem Endstück abgeschlossenen Modul zugehört.

5. Sonde nach Anspruch 4, dadurch gekennzeichnet, dass der Lichtsender (82) und das Objektiv (83) des Endoskops (80) an der distalen Stirnfläche des letzten angeordnet sind und dass das Endstück Spiegel enthält, die hinter dem Wandler angeordnet sind und dazu dienen, die Beobachtung mit Hilfe des Endoskopes zu ermöglichen.

6. Sonde nach Anspruch 4, dadurch gekennzeichnet, dass das Objektiv (83) und der Lichtsender (82) des Endoskops am distalen Endteil des Endoskop-Moduls auf einer seitlichen und bezüglich der Achse des Endoskops geneigten Oberfläche angeordnet sind.

7. Sonde nach einem der vorhergehenden Ansprüche, dadurch gekennzeichnet, dass das Endstück eine gedruckte Schaltung (26) enthält, die mittels dünner Verbindungsdrähte (37) mit den piezo-elektrischen Elementen des Wandlers (25) und mittels Miniatur-Koaxialkabel mit einem elektronischen Gerät an dem dem Endstück abgekehrten Ende des rohrförmigen Organs verbunden ist.

8. Sonde nach einem der vorhergehenden Ansprüche, dadurch gekennzeichnet, dass der Wandler zur Fokussierung in Richtung quer zur Symmetrieebene der Sonde eine konvexe akustische Linse aufweist, welche aus einem Material besteht, in dem die Fortpflanzungsgeschwindigkeit von Ultraschall kleiner ist als in biologischen Geweben.

**Claims**

1. Endoscopic probe comprising a tubular member adapted to be inserted into a cavity to be explored, having a flexible part provided with a distal section whose angular position is adjustable by mechanical control means located within the flexible part, illuminating and image-returning optical means (18, 19, 20, 23) and an electro-acoustic transducer (25) carried by said section and having a linear array (31) of piezo-electric elements fixed in the section and associated with an electronic circuit for energization of the elements according to a scanning sequence within a field having an overlap with the field of view of the optical means, characterized in that the array is curved, the convexity of the curve being directed outwardly, and in that the electronic circuit has electric delay and/or phase shift means for achieving an electronic sector scan within a plane which passes through the axis of the distal section (14) of the probe by energization of successive groups of piezo-electric elements with focussing at a predetermined distance from the array.

2. Probe according to claim 1, characterized in that the electronic circuit has means for modifying the distribution of the phases and/or delays during successive bursts from a same group of piezo-electric elements so as to provide scanning along lines additional to lines normal to the elements.

3. Probe according to claim 1 or 2, characterized in that the optical means are disposed at the end of the distal section, which is flexible and angularly adjustable, beyond said electro-acoustic transducer, and in that the mechanical means are so arranged as to give to said distal section, during use of the endoscope, a curved shape such that there is an overlap of the field of view of the optical means and of the angular sector scan field of the transducer.

4. Probe according to claim 1 or 2, characterized in that it consists of two modules (80, 81) which are separate and insertable into each other, one of which is formed by an endoscope having said optical means, insertable in a sheath (86) belonging to the other module which ends in said distal section.

5. Probe according to claim 4, characterized in that the light emitter (82) and the objective lens (83) of the endoscope (80) are disposed in a distal end surface of the latter and in that said distal section comprises mirrors placed behind said transducer and adapted for authorizing observation with the endoscope.

6. Probe according to claim 4, characterized in that the objective lens (83) and the optical emitter (84) of the endoscope are placed at the distal end of the endoscopic module, laterally and on a surface slanting with respect to an axis of the endoscope.

7. Probe according to anyone of the preceding claims, characterized in that said distal section contains a printed circuit (26) connected by thin junction wires (37) to the piezo-electric elements of the transducer (25) and by miniature coaxial cables to an electronic box connected to the end of the tubular part which is farthest from the distal section.

8. Probe according to anyone of the preceding claims, characterized in that the transducer comprises a convex acoustic lens for focusing in a direction transverse to a plane of symmetry of the probe, of a material in which the propagation velocity of the ultra sounds is less than that in biological tissues.

Fig.1.

Fig.2.

Fig.3.

Fig.4.

Fig.5.

Fig.6.

Fig.7.

0 123 594

Fig.8.

Fig.9.

Fig.10.

Fig.11.